# EUROPÄISCHE PATENTSCHRIFT

(11) **EP 2 717 963 B1**
(45) Veröffentlichungstag und Bekanntmachung des Hinweises auf die Patenterteilung: **11.01.2017**
(21) Anmeldenummer: 12720900.5
(22) Anmeldetag: 16.05.2012
(51) Int. Cl.: A61N 5/10

(54) **SCHICHTAUFBAU ZUR EPIDERMALEN RADIONUKLIDTHERAPIE**
MULTILAYER STRUCTURE FOR EPIDERMAL RADIONUCLIDE THERAPY
STRUCTURE EN COUCHES POUR THÉRAPIE RADIONUCLÉAIRE ÉPIDERMIQUE

(30) Priorität: 06.06.2011 DE 102011050848
(43) Veröffentlichungstag der Anmeldung: 16.04.2014
(73) Patentinhaber: OncoBeta International GmbH, 88214 Ravensburg (DE)
(72) Erfinder: DESANTIS, Maria, I-00192 Rom (IT); BUCK, Oliver, 83457 Bayerisch Gmain (DE); CIPRIANI, Cesidio, I-00185 Rom (IT)
(74) Vertreter: Winter, Brandl, Fürniss, Hübner, Röss, Kaiser, Polte - Partnerschaft mbB
(86) Internationale Anmeldenummer: PCT/EP2012/059108
(87) Internationale Veröffentlichungsnummer: WO 2012/168047

(56) Entgegenhaltungen:
- EP-A1- 2 098 251
- DE-A1- 3 133 909
- FR-A- 1 332 345
- US-B1- 6 350 226

## Beschreibung

Die vorliegende Erfindung betrifft einen Schichtaufbau zur epidermalen Radionuklidtherapie eines Patienten gemäß Anspruch 1.

Hautkrebs ist die häufigste Form von Krebserkrankungen, die beim Menschen vorkommen, und betrifft in manchen Ländern bis zu 50 % sämtlicher Tumorarten. Unter sämtlichen beim Menschen vorkommenden Tumoren ist das Basalzellkarzinom (BCC) die häufigste innerhalb der weißen Population auftretende Krebserkrankung. Etwa 80 % der Hautkrebsfälle sind Basalzellkarzinome. BCC ist ein langsam wachsender, lokal invasiver maligner epidermaler Hauttumor. Er tendiert dazu, das Bindegewebe zu infiltrieren und zu zerstören, jedoch ist eine Metastasierung sehr selten. Zu Beginn der Erkrankung handelt es sich um einen oberflächlichen transluzenten Knoten mit wachs- oder perlgrauer Farbe. Die am meisten fortgeschrittenen Formen zeigen häufig Ulzerationen auf, insbesondere in ihrem mittleren Bereich und an den Rändern. Basalzellkarzinome können an jeglicher Körperstelle auftreten, jedoch erscheinen 90 % der Läsionen im Gesicht und am Kopf. Das BCC kommt am häufigsten bei hellhäutigen Patienten mittleren bis gehobenen Alters vor, die in ihrer Anamnese eine Belastung mit ultravioletter Strahlung angeben, jedoch können Basalzellkarzinome auch in einem Basalzellnävus als sogenanntes Gorlin's Syndrom auftreten. Aufgrund der Störungen in der Ozonschicht hat Australien das höchste BCC-Vorkommen weltweit, und in manchen Regionen liegt die Tumorinzidenz pro Jahr bei bis zu 2 %.

Wenn sich bei einem Patienten ein BCC entwickelt hat, besteht ein signifikant erhöhtes Risiko, dass sich weitere Basalzellkarzinome an anderen Stellen des Körpers entwickeln. Untersuchungen haben gezeigt, dass das Basalzellkarzinom aus multipotenten Zellen in der Basalschicht oder den Follikeln der Haut entstehen kann. Es gibt jedoch unterschiedliche histologische und pathologisch klinische Formen des Basalzellkarzinoms, jedoch liefern die herkömmlichen diagnostischen Verfahren nicht genug Information bezüglich der Tumoreigenschaften.

Der zweithäufigste auftretende Hauttumor ist neben dem Basalzellkarzinom das Plattenepithelkarzinom (squamous cell carcinoma, SCC). Das SCC ist eine maligne epitheliale Erkrankung mit morphologischen Eigenschaften der Plattenzelldifferenzierung ohne zusätzliche Merkmale, die andere differenzierte Gewebe nahelegen. Es kann an jedem Körperbereich auftreten und kann sich ebenfalls an den Lippen, der Vulva und dem Penis entwickeln. Es wurde häufig beobachtet, dass das Plattenepithelkarzinom als Folge von Verbrennungsnarben oder Ulzera entsteht und als eine oberflächliche Läsion erscheint, welche leicht blutet. Manchmal entwickelt sich eine Ulzeration mit dicken kraterähnlichen Rändern. In anderen Fällen ist die Läsion mit Hornschichten überdeckt.

Andere Varianten dieser Tumorart entstehen aus der oberflächlichen Schicht und sind insbesondere als Bowen's Dermatose bekannt und entsprechen einem Stachelzellkrebs *in situ*, welcher dann möglicherweise erst nach weiteren Jahren zu einem Durchbruch durch die Basalschicht und Eindringen in das Korium zu einem verhornenden Plattenepithelkarzinom führt. Eine Präkanzerose für das Plattenepithelkarzinom stellt auch die sogenannte Queyrat-Erythroplasie dar, welche eine oberflächliche Form eines *in situ-*SCC der männlichen und weiblichen Genitalbereiche sowie von Lippen und Mund darstellt. Bei der Erythroplasie handelt sich um relativ scharf begrenzte, wenig infiltrierte, feucht glänzende oder erosive Veränderungen.

Eine besondere Form des Plattenepithelkarzinoms ist das Keratoakanthom, welches eine Hautgeschwulst ist, die vermutlich von den Haarfollikeln ausgeht.

Schlussendlich tritt als Lichtschaden auch die aktinische Keratose, auch solare Keratose genannt, auf, welche heutzutage in der Dermatologie als beginnende Form eines Carcinoma *in situ* gilt.

Wie bereits oben erwähnt, ist das Plattenepithelkarzinom die zweithäufigste Hautkrebsform mit über 200.000 neuen Fällen pro Jahr in den USA. Nicht überraschend tritt die Erkrankung am häufigsten in Australien auf, wobei eine alterskorrigierte Inzidenz von 1332 Fällen pro 100.000 Einwohner für Männer und 755 Fällen pro 100.000 Einwohner für Frauen berechnet wurde. In den europäischen Ländern beträgt die jährliche Inzidenz des Plattenepithelkarzinoms derart etwa 25 Fälle pro 100.000 Einwohner. Das Plattenepithelkarzinom der Haut kann in die regionalen Lymphknoten metastasieren und rezidiviert häufig lokal.

Das Auftreten von BCC und SCC wächst mit zunehmendem Alter und Beginn etwa bei 30 Jahren und weist einen Altersgipfel zwischen 65 und 70 Jahren auf. Die Erkrankungen treten bei Männern häufiger auf als bei Frauen. Beide Tumorarten erscheinen am häufigsten im Gesicht, dem Halsbereich, der haarlosen Kopfhaut, den Händen, Schultern, Armen und am Rücken. Die Ränder der Ohren und der Unterlippe sind besonders häufig von diesen Krebsarten betroffen. Die klinischen Erscheinungsformen und Morphologien beider Tumorarten sind unterschiedlich und schließen knotenbildende, zystische ulzerierte (rodent ulcer), oberflächliche, morphoische (sklerosierende), keratotische und pigmentierte Varianten ein.

Die Ulzeration tritt besonders häufig bei großen Tumoren auf, die schon lange bestehen, oder bei aggressiven Läsionen. Die Risikofaktoren für beide Tumorarten schließen Sonnenexposition, Exposition mit ionisierender Strahlung, Arsenexposition, Teerderivate- und UVA-Strahlungsexposition ein. Es wurde ebenfalls festgestellt, dass gewisse prädisponierende Faktoren eine Rolle spielen, wie beispielsweise Immunsuppression, physikalische Charakteristika, Hautkolorit, rote oder blonde Haare und helle Augenfarbe.

Während das Basalzellkarzinom eher durch ein nicht aggressives Verhalten gekennzeichnet ist, was an seiner relativ niedrigen Metastasierungsrate von etwa 0,03 bis 0,6 % liegt (Metastasen wurden jedoch beschrieben im Unterhautgewebe, den Knochen, der Lunge, der Leber und den Lymphknoten des Halsbereichs), weist das Plattenepithelkarzinom eine deutlich aggressivere Verlaufsform auf, und dessen Metastasierungspotenzial ist deutlich höher und liegt bei etwa 2 bis 5 %. Aufgrund der nur schwer möglichen alleinigen makroskopischen und morphologischen Begutachtung von Läsionen durch einen erfahrenen Dermatologen ist die korrekte Diagnose von fundamentaler Bedeutung. Diese wird jedoch nur ermöglicht durch die mikroskopische, histologische und zytologische Untersuchung, mit welcher eine exakte Charakterisierung und Klassifikation durchgeführt werden kann. Gewebeproben können entnommen werden durch chirurgische Exzision oder Biopsie mit geeigneten Biopsiestanzen. Manchmal genügt auch eine einfache zytologische Untersuchung der vernarbten Läsion, um eine Verdachtsdiagnose zu bestätigen. Die dermoskopische Epilumineszenz, welche in großem Bereich für die Diagnose von pigmentierten Läsionen verwendet wird, erlaubt die Beobachtung und Charakterisierung von Merkmalen und die Bewertung des Gefäßverlaufs innerhalb der Läsion. Die Beobachtung von Neoangiogenesen, welche die Krebsläsion zeichnen, liefert in der Regel nützliche Hinweise für die Beurteilung der Ausdehnung und der Tiefe der Läsion. Aufgrund des hohen Risikos einer metastatischen Dissemination ist eine genaue zeitliche Überwachung des Patienten zwingend erforderlich.

Klinische Untersuchungen erfordern eine Ganzkörperhautuntersuchung, Palpation der chirurgisch behandelten Stellen und eine Untersuchung der Haut zwischen Primärtumorstellen und ableitenden Lymphknoten für eine *in transit-*Metastasierung*.* Regionale Lymphknoten sollten auf eine Lymphadenopathie abgetastet werden, und verdächtige Lymphknotenvergrößerungen sollten mittels Biopsie oder bildgebender Verfahren oder beiden untersucht werden. Besonders nützliche bildgebende Techniken sind die Verwendung des Computertomogramms oder Computertomogramm in Verbindung mit Positronenemissionstomographie. Magnetresonanzuntersuchungen liefern die beste Auflösung für Weichgewebetumoren, insbesondere in Kopf- und Halsbereich, und sollten erwägt werden zum Screenen von Metastasen, die in diesen Bereichen auftreten könnten. Sollten verdächtige Lymphknoten auftreten, so ist eine Überwachung der Lymphknotenlokalisation und Lymphoszintigrafie mittels ^{99m}Tc-Kolloid vor und nach der Therapie in allen Verdachtsfällen in Erwägung zu ziehen. Charakteristiken von Primärtumoren, welche sich zu metastatischen Plattenepithelkarzinomen entwickeln können, schließen eine Fläche von >120 mm², eine Invasionstiefe von >3,2 mm und eine Invasion in darunterliegendes Fett-, Muskel- oder Knochengewebe ein.

Als primäres Mittel der Wahl bietet sich zunächst die chirurgische Intervention an, wobei Ränder von 2 bis 4 mm für noduläre, gut abgegrenzte Tumoren bis zu einer Größe von 2 cm empfohlen werden. Für Tumoren, die größer als 2 cm sind, wird eine Exzision mit einem Rand von 1 cm oder mehr im Allgemeinen vorgeschlagen, insbesondere für Tumoren mit aggressivem Verlauf.

Als chirurgisches Verfahren hat sich Mohs'-Technik als erfolgversprechendstes Verfahren für Heilung bei gleichzeitiger Erhaltung von gesundem Gewebe herausgestellt. Es besteht in einer progressiven histologischen Echtzeituntersuchung der Gewebeschnitte der Läsion während der Chirurgie, bis man das gesunde Gewebe erreicht.

In sämtlichen Fällen, in denen Tumoren in Bereichen auftreten, die durch chirurgische Intervention schwer zugänglich sind, wie beispielsweise Ohren, Nase und Augenlider, sind die ästhetischen und funktionellen Resultate der chirurgischen Exzision häufig sehr unbefriedigend. Wenn die Läsion eher groß ist und die verbleibende gesunde Haut nicht für eine zufriedenstellende chirurgische Naht ausreicht, ist es erforderlich, zu Maßnahmen der plastischen rekonstruktiven Chirurgie zu greifen, wobei Hauttransplantationen (gewöhnlich aus den unteren Extremitäten oder von der Gluteushaut) unvermeidlich sind.

Das kosmetische Ergebnis dieser Maßnahmen ist jedoch häufig nicht zufriedenstellend. Falls ein Rezidiv innerhalb der transplantierten Haut auftritt, wird das Behandeln der Läsionen extrem problematisch.

Für beide Tumorarten gibt es Standardtherapien wie die Kürettage und Elektrodessikation; Chirurgie, Kryochirurgie und intraläsionale Interferontherapie werden den Patienten häufig vorgeschlagen.

Mittlerweile stehen auch neuere topische Therapien zur Behandlung ausgewählter Fälle zur Verfügung, wie beispielweise bei Tumoren, die in kritischen Bereichen auftreten, oder bei inoperablen Patienten, die andere systemische Erkrankungen wie beispielsweise Kardiomyopathie, pulmonäre Insuffizienz usw. aufweisen, so dass eine chirurgische Intervention unter Allgemeinanästhesie kontraindiziert ist. Diese Therapieformen schließen die Behandlung mit Imiquimod, einem Virustatikum und Immunmodulator, ein, welche zur Behandlung des oberflächlichen BCC, aktinischer Keratose und Feigwarzen (Condylomata acuminata) eingesetzt wird. Tazarotene, eine Rezinsäure, welche im Allgemeinen zur topischen Behandlung von Psoriasis verwendet wird, wurde vorgeschlagen zur lokalen Behandlung von BCC. Darüber hinaus wurde eine fotodynamische Therapie vorgeschlagen, die das Anwenden eines tumorlokalisierenden, fotosensitivierenden Mittels und dessen nachfolgende Aktivierung mit sichtbarem Licht beinhaltet, um eine selektive Zerstörung des Tumors zu bewirken. Die Verwendung von Imiquimod-Creme als eine wirksame Behandlungsoption für oberflächliche und noduläre Basalzellkarzinome wies eine Erfolgsrate von 89,5 % bei einer durchschnittlichen 39-monatigen Folgekontrolle auf (Vun Y, Siller G, Australas J Dermatol. 2006, Aug; 47(3): 169-71). Die Verwendung einer fotodynamischen Therapie mit Porfimer-Natrium wurde ebenfalls beschrieben.

Darüber hinaus sind fotodynamische Therapien mit meso-Tetrahydroxyphenylchlorinen (m-THPC) aus dem Stand der Technik bekannt.

Sämtliche genannten Behandlungsverfahren werden verwendet, um kleine, oberflächliche und nicht rezidivierende BCC zu behandeln, sind jedoch nicht indiziert für noduläre, zystische, infiltrative und morphoische Varianten des Basalzellkarzinoms.

Darüber hinaus wurde im Stand der Technik versucht, über eine Photonenbestrahlung mit Dosen von 20 bis 73 Gy in einzelnen oder mehrfachen Behandlungen BCC zu behandeln. Bei diesem Behandlungsverfahren traten in einem Fünfjahreszeitraum bei Stufe I- und II-Karzinomen in 95 % der Fälle keine Rezidive auf. Somit können die Erfolge einer Bestrahlungstherapie hinsichtlich ihrer Heilungsraten vergleichbare Ergebnisse liefern wie die Mohs' mikrografische Chirurgie, welche bei diesen Tumoren im Allgemeinen als "Goldstandard"-Behandlung angesehen wird.

Eine Bestrahlung mit konventionellen Methoden, insbesondere eine Radiotherapie durch Anwenden von externen Röntgen- oder γ-Strahlen aufgrund der Durchdringungseigenschaften der Photonen kann nicht für die Behandlung von Tumoren in solchen Bereichen empfohlen werden, wo sie sehr schädlich, zum Beispiel für Gesicht und Augen, sein kann. Darüber hinaus ist die Röntgen- und γ-Bestrahlung des Plattenepithelkarzinoms auch nicht erfolgversprechend für die Behandlung geeignet.

Ein erster vielversprechender Ansatz zur Behandlung des Basalzellkarzinoms und des Plattenepithelkarzinoms ist in Sedda AF, Rossi G, Cipriani C, Carrozzo AM und Donati T, 2008: Dermatological high-dose-rate brachytherapy for the treatment of basal and squamous cell carcinoma, Clinical and Experimental Dermatology, 33, 745-749 sowie in EP 2 098 251 A1 beschrieben.

Andere Beispiele findet man in US 6350226 und FR 1332345. Das Dokument US 6350226 offenbart ein Schichtaufbau zur epidermalen Radionuklidtherapie mit Merkmalen des Oberbegriffs des Anspruchs 1.

In dem genannten Stand der Technik wird ein Behandlungsverfahren für das Basalzellkarzinom sowie für das Plattenepithelkarzinom beschrieben, bei welchem radioaktive β-Strahlen-emittierende Isotope eingesetzt werden, insbesondere ¹⁸⁸Re.

In dem beschriebenen Verfahren wird die zu behandelnde Hautläsion mit einer dünnen Schicht einer Schutzcreme überstrichen und auf die Schutzcreme eine radioaktive Matrix aus ¹⁸⁸Re₂S₇ in Form eines Kolloids, welcher gründlich mit einem synthetischen Acrylharz vermischt ist, auf die Hautcremeschicht aufgetragen.

Die Polyacrylatschicht härtet ohne wesentliche Schrumpfung aus und wird je nach Behandlungsschema zwischen 15 Minuten und 3 Stunden auf der zu behandelnden Läsion belassen.

Im Anschluss daran wird die ausgehärtete ¹⁸⁸Rhenium-Acrylharzdispersion manuell wieder von der Läsion entfernt.

Hierbei haben sich ¹⁸⁸Re-Isotope als besonders nützlich herausgestellt, weil ¹⁸⁸Re ein gemischter β-γ-Emitter ist mit einer Halbwertszeit von etwa 16,98 Stunden. Die emittierten β-Partikel haben eine maximale Energie von etwa 2,12 MeV und eine mittlere Energie von 764 KeV und sind somit nur über eine kurze Distanz therapeutisch wirksam. Der γ-Strahlenanteil beträgt etwa 15 % der Strahlungsintensität und hat eine Energie von etwa 155 KeV.

Darüber hinaus ist die γ-Strahlung hervorragend geeignet, um etwaige radioaktive Kontaminationen mit ¹⁸⁸Re zu erfassen.

Bei Untersuchungen hat sich gemäß der EP 2 098 451 A1 herausgestellt, dass die Eindringtiefe für die β-Strahlung etwa 200 bis 600 µm in die Epidermis beträgt, was selbstverständlich von den histologischen Gegebenheiten abhängt. Die Aktivität der β-Emitterisotope, welche in der Behandlung gemäß EP 2 098 451 A verwendet wurden, lag im Bereich von 3,7 x 10⁻² Gbq bis 1,85 Gbq bei größeren Läsionen. Neben ¹⁸⁸Re wurden auch Verbindungen von ⁹⁰Y, ³²P und ¹⁶⁶Ho verwendet.

In einem Patientenkollektiv mit 200 histologisch gesicherten Diagnosen von BCC oder SCC reichte in der Regel bei 85 % der behandelten Patienten eine einzige Behandlung zur vollständigen klinischen Remission aus, die nach 3-5 Monaten einsetzte.

Es wurden gemäß einer Follow-up-Studie von im Mittel vier Jahren insgesamt Heilerfolge mit bis zu 100 % erreicht.

Demzufolge ist die Brachytherapie mit β-Strahlung und insbesondere ¹⁸⁸Re ein vielversprechender therapeutischer Ansatz bei solchen BCC- und SCC-Läsionen, die chirurgisch nur sehr schwer und mit kosmetisch störenden Effekten angegangen werden können.

Da jedoch die Dosis sehr genau berechnet werden muss, welche für die Behandlung einer speziellen Läsion erforderlich ist, und die Läsionen zudem geometrisch häufig komplex ausgebildet sind, ergab sich aufgrund der von Ort zu Ort unterschiedlicher Tiefe der zu behandelnden Läsion eine unterschiedliche Füllung mit Schutzcreme, welche zu einer Ortsabhängigkeit der Intensität der β-Strahlung - und damit der ß-Exposition - führte, da die Strahlung bei größerer Menge an Schutzcreme stärker abgeschwächt wird als in Läsionsbereichen, die flacher verlaufen und dementsprechend weniger Schutzcreme enthalten oder gar Erhebungen aufweisen.

Darüber hinaus ergab sich ein weiteres Problem aus der Entfernung der radioaktiven Polyacrylatmatrix von der Hautoberfläche des Patienten, da zum einen mit Instrumenten zwischen Polyacrylatschicht und Hautoberfläche in die Cremeschicht eingegriffen werden musste und es hierbei manchmal zu Rissen der radioaktiven Schicht mit möglicherweise sanfter radioaktiver Kontamination der Cremeschicht kam.

Dies führte zu aufwendigen Nachbehandlungen, bei denen die Läsion vollständig dekontaminiert werden musste, was einerseits das Einsetzen der Heilung verzögerte und andererseits mit weiteren Unannehmlichkeiten des Patienten einherging.

Ausgehend vom Stand der Technik der EP 2 098 251 A1 ist es daher die Aufgabe der vorliegenden Erfindung, eine weitgehend gleiche Bestrahlung über die gesamte Läsion unter Vermeidung unterschiedlicher Abschwächungen der β-Strahlung im Bereich der Hautläsionen zu gewährleisten und gleichzeitig dafür Sorge zu tragen, dass die radioaktive Schicht leicht wieder vollständig ohne jegliche Kontamination von der Hautoberfläche des Patienten entfernt werden kann.

Die Lösung dieser Aufgabe erfolgt durch die Merkmale des Patentanspruchs 1.

Insbesondere betrifft die Erfindung einen Schichtaufbau zur epidermalen Radionuklidtherapie eines Patienten, mit:
- einer, aus Patientensicht proximalen, Haftschicht zum Anbringen des Schichtaufbaus auf einer zu behandelnden Hautoberfläche des Patienten, wobei eine Haftschicht aus einer Hautcreme und/oder einem Hautgel ausgenommen ist;
- einer aus Patientensicht in distaler Richtung über der Haftschicht angeordneten flexiblen, transparenten Trägerschicht; sowie
- wenigstens einer auf der Trägerschicht angeordneten radionuklidhaltigen Emissionsschicht;
wobei die Haftschicht und die Trägerschicht hinsichtlich ihrer Bestandteile und Dicke so ausgebildet sind, dass sie für γ-Strahlung und im Wesentlichen für β-Strahlung durchlässig sind. Bei Bedarf können die Haft- und Trägerschichten hinsichtlich ihres Materials und Dicke auch so dünn ausgebildet werden, dass sie für α-Strahlung, z.B. mit 4 bis 6 MeV, durchlässig ist, wozu Schichtdicken von deutlich unter 50 µm und Dichten ≤ 1 g/cm³ erforderlich sind.

Ein vorteilhafter Schichtaufbau ist ein solcher, bei welchem die Haftschicht vor dem Zusammenbau des Schichtaufbaus auf der Hautoberfläche gebildet wird, wobei die Haftschicht z.B. aus einem auf die Hautoberfläche aufsprühbaren Kunststofffilm gebildet ist, insbesondere einem Sprühverband auf Basis von Polyurethanen, Polyacrylaten, insbesondere Poly(butylmethacrylat,methylmetacrylat); oder 2-Octyl-cyanacrylat. Durch diese Maßnahme können zwei Probleme gleichzeitig gelöst werden: Zum Einen erfolgt durch die Überziehung der Läsion mit einer derartigen separaten Haftschicht ein oberflächlicher Verschluss, der auch beispielsweise gegen Infektionen schützt und gleichzeitig dient die Haftschicht zur Ausrichtung und Fixierung der Trägerschicht. Eine Transparenz von Haftschicht und Trägerschicht ist von großem Vorteil, da der behandelnde Arzt sowohl die Läsion als auch die Hautmarkierungen zum Eingrenzen des zu behandelnden Bereiches durch die Schichten hindurch erkennen kann.

Typische Haftschicht-Sprühfilme weisen eine Dicke von 1 bis 80 µm, insbesondere von 3 bis 60 µm auf. Dickenmessungen haben überraschend ergeben, dass die Dicke dieser aufgesprühten Haftschichten relativ konstant sind und nur kleineren Schwankungen unterliegen, die therapeutisch irrelevant sind.

Gemäß dem erfindungsgemäßen Schichtaufbau ist die Haftschicht auf der proximalen Seite der Trägerschicht angeordnet.

Insbesondere bei kleineren und geometrisch wenig anspruchsvollen Hautläsionen hat sich eine Trägerschicht als vorteilhaft herausgestellt, die zugleich Haftschicht ist, wobei die Trägerschicht als selbsthaftende Schicht, etwa nach Art einer handelsüblichen einseitig beschichteten Klebefolie ausgebildet ist.

Für die vorliegende Erfindung kommen Trägerschichten in Form von Folien in Betracht, die eine Dicke von 15 bis 80 µm, insbesondere von 20 bis 60 µm, vorzugsweise von 30 bis 40 µm, aufweisen. Derartige Trägerschichten sind hinreichend dünn, um sich jeder komplizierten Geometrie einer Läsion wie beispielsweise im Helixbereich der Ohrmuschel, dem Augenbereich oder in der Nasolabialfalte sowie auch an äußeren und inneren Labien der Vulva und am Penis im Wesentlichen anzupassen.

Bevorzugte Materialien für die Trägerschicht-Folie werden ausgewählt aus der Gruppe bestehend aus: Polyurethanfolie, Polyamidfolie, Polyolefinfolie, insbesondere Polyethylen- und Polypropylenfolie. Diese Kunststoffe weisen den Vorteil der Biokompatibilität bei gleichzeitiger Sterilisierbarkeit und Verfügbarkeit zu geringen Kosten auf. Darüber hinaus lassen sie die Elektronen der ß-Strahlung bei den im erfindungsgemäßen Schichtaufbau eingesetzten Schichtdicken praktisch ungehindert durch.

Für das Abziehen des Schichtaufbaus von der Hautoberfläche bzw. von der Haftschicht ist es von Vorteil, wenn die Trägerschicht-Folie in ihrem äußeren Randbereich wenigstens eine Zone ohne Haftkraft aufweist. Dort kann nach Abschluss der Behandlung die Trägerschicht-Folie bequem mit einer speziellen Zange, etwa einer solchen gemäß DE 20 2010 005 805 U1 zusammen mit der Emissionsschicht und einer etwaigen Abdeckschicht abgezogen und fachgerecht entsorgt werden.

Typischerweise ist bei dem erfindungsgemäßen Schichtaufbau die Emissionsschicht eine Polymermatrix, wobei eine wasserbasierte Polyacrylatmatrix bevorzugt ist. Derartige Polymermatrices sind aus dem Polyacrylatfarbenbereich bekannt und daher mit sämtlichen gewünschten Eigenschaften wie Farbe, Viskosität, Verarbeitungszeit, Aushärtetemperatur usw. verfügbar. Sie enthalten ferner keine organischen Lösungsmittel, die Patient und medizinisches Personal belasten könnten.

Der Schichtaufbau der vorliegenden Erfindung enthält gemäß einer bevorzugten Ausführungsform eine Emissionsschicht, die ein Radionuklid enthält, welches ausgewählt ist aus der Gruppe bestehend aus: ß-Emittern, insbesondere ³²P, ⁹⁰Y, ¹⁶⁶Ho, ¹⁷⁷Lu, und ¹⁸⁸Re.

Diese Radionuklide haben sich in der Therapie des BCC und des SCC bestens bewährt und sind zudem relativ leicht verfügbar.

Als besonders vorteilhaft hat sich erwiesen, dass die Emissionsschicht eine Polyacrylatmatrix mit homogen darin verteiltem ¹⁸⁸Rheniumsulfid, z.B. ¹⁸⁸Re₂S₇ ist. Diese Verbindung bildet ein Kolloid und gewährleistet somit eine extrem homogene Verteilung und gleichzeitig eine stabile Dispersion, so dass eine homogene Strahlungsquelle in der Emissionsschicht zur Verfügung steht. Zur Zeit ist es möglicherweise nicht ganz geklärt, ob Rheniumsulfid ausschließlich als Re₂S₇ vorliegt oder ob möglicherweise weitere Sulfide, auch in gemischter Form, vorliegen können.

Daher sollen für die Zwecke der vorliegenden Erfindung unter der Formel ¹⁸⁸Re₂S₇ auch möglicherweise weitere Sulfide - auch etwaige Mischsulfide - des Rheniums, verstanden werden, die somit ebenfalls in den Schutzbereich des vorliegenden Patentes/Anmeldung fallen.

Bei Bedarf kann der Schichtaufbau nach außen durch eine distal auf der Emissionsschicht angeordnete Abdeckschicht abgeschlossen werden. Hierdurch wird die Emissionsschicht "sandwichartig" durch Trägerschicht und Abdeckschicht abgeschlossen. Diese Maßnahme gewährleistet einerseits eine weitere Abschirmung für das medizinische Personal und andererseits verhindert sie auch die versehentliche Kontamination durch unruhige und unachtsame Patienten, die durch unbedachte Bewegungen mit der Emissionsschicht in Berührung kommen können und sich selbst und die nuklearmedizinische Behandlungseinheit kontaminieren können.

Typischerweise ist die Abdeckschicht hinsichtlich ihrer Beschaffenheit und Dicke so ausgebildet ist, dass sie für die von den Radionukliden der Emissionsschicht emittierte α-, ß- und γ-Strahlung undurchlässig ist.

Als vorteilhafte Materialien für die Abdeckschicht haben sich eine Polyester-, Polyethylen- oder Polypropylen-Folie oder eine mit Polyester, Polyethylen oder Polypropylen laminierte Aluminiumfolie herausgestellt, welche vorzugsweise eine Dicke von 100 bis 200 µm aufweisen.

Die Abdeckschicht wird bevorzugt auf die noch nicht vollständig ausgehärtete Emissionsschicht aufgebracht und haftet nach dem Aufbringen auf der Emissionsschicht.

Mit den Radionukliden ³²P, ⁹⁰Y, ¹⁶⁶Ho, ¹⁷⁷Lu und ¹⁸⁸Re stehen leicht verfügbare β-Emitter zur Verfügung. So kann ¹⁸⁸Re beispielsweise über den dem Fachmann wohlbekannten ¹⁸⁸W/¹⁸⁸Re-Generator erzeugt werden. ¹⁸⁸Re weist zudem eine relativ kurze Habwertszeit von 16,7 h auf, so dass anfallender radioaktiver Abfall rasch abklingt und nicht zu Entsorgungsproblemen führt.

Durch die Maßnahme, dass der erfindungsgemäße Schichtaufbau in distaler Richtung auf der Emissionsschicht eine Abdeckschicht aufweist, ist sichergestellt, dass die radionuklidhaltige Emissionsschicht quasi in sich geschlossen ist, so dass beim Abnehmen keinerlei radioaktive Kontaminationen weder auf Patientenseite noch auf Seiten des medizinischen Personals zu befürchten sind.

Als besonders vorteilhaft hat sich hierbei eine Abdeckschicht aus einer Polyester-, Polyethylen- oder Polypropylenfolie herausgestellt oder aber eine polyester-, polyethylen- oder polypropylenlaminierte Aluminiumfolie mit Dicken von etwa 100-200 µm.

Weitere Vorteile und Merkmale der vorliegenden Erfindung ergeben sich aufgrund der Beschreibung eines Ausführungsbeispiels.

### Ausführungsbeispiel

### Vorbereitung des Patienten

Vor der Behandlung mit dem ausgewählten Radionuklid, im Beispielsfall ¹⁸⁸Re, muss die Läsion von einem Dermatologen untersucht werden und der zu behandelnde Bereich festgelegt werden. Dieser wird dann mit einem hautfreundlichen Stift angezeichnet. Die Fläche des angezeichneten Bereichs, der mit dem radioaktiven Nuklid behandelt werden soll, muss bestimmt werden. Dies geschieht durch computerunterstütztes Auswerten eines Fotos oder eines Scans des markierten zu behandelnden Bereichs.

Zur Behandlung wird der Patient in eine Position gebracht, die einen weitgehend waagrechten Auftrag der flüssigen Matrix ermöglicht, so dass sie nicht verläuft. In der Regel liegt der Patient dabei auf einer Liege, und es ist außerdem darauf zu achten, dass die Haltung für den Patienten angenehm ist, da er in dieser Position 1-3 Stunden verharren muss.

Die Berechnung der ß-Strahlungsdosis für das ausgewählte Radionuklid - hier ¹⁸⁸Re - erfolgt nach an sich bekannten Methoden.

Die zu behandelnde Hautläsion muss vor der Behandlung gesäubert und trockengelegt werden. Eventuell vorhandener Schorf ist vorsichtig zu entfernen. Auch darf die Läsion nicht bluten oder Sekrete abgeben. Für den Fall, dass Blutungen auftreten, werden geeignete, an sich bekannte Blutstillungsmaßnahmen verwendet.

Auf die Läsion selbst und auf die Haut im Umfeld wird ein flüssiges Sprühpflaster auf Polyurethanbasis als Haftschicht unmittelbar auf die Hautläsion aufgetragen. Abschließend wird ein paar Sekunden gewartet, bis keine Lösungsmittel mehr wahrgenommen werden können.

Messungen an derartigen aufgesprühten Haftschichten haben ergeben, dass sie eine Dicke in einem Bereich von etwa 3-60 µm aufweisen und entgegen der allgemeinen Erwartungen nur relativ geringe Schichtdickenschwankungen zeigen.

Auf die aufgetragene Haftschicht wird eine sterile, sehr dünne, ca. 35 µm dicke, transparente medizinische Polyurethanfolie auf die Haut mit der aufgetragenen Haftschicht aufgelegt und vorsichtig angedrückt. Diese Polyurethanfolie dient als Trägerschicht. Die Trägerschicht-Folie klebt an der Haftschicht fest und wird somit fixiert. Durch Beschaffenheit und Dicke im zweistelligen µm-Bereich wird es ermöglicht, dass sich die Trägerfolie faltenfrei auch komplex geometrischen Läsionen, wie etwa am Randbereich der Helix auriculae oder in der Nasolabialfalte anpasst und dabei gleichzeitig einen im Wesentlichen gleichen Abstand der Läsionsoberfläche zur (patientenseitig) proximalen Seite der Emissionsschicht gewährleistet. Hierdurch wird gleichzeitig eine gleiche Strahlendosis für sämtliche Bereiche der zu behandelnden Läsion sichergestellt.

Die Trägerschicht-Folie wird so auf die Haut geklebt, dass am Rand Bereiche lose bleiben, die nicht mit Haftschicht verbunden sind, damit die Trägerschicht-Folie dort später mit Hilfe einer speziell ausgestalteten Zange (etwa gemäß dem deutschen Gebrauchsmuster DE 20 2010 005 805 U1) gegriffen und der gesamte Schichtaufbau von der Haftschicht und damit der Läsion abgezogen werden kann. Die Trägerschicht sollte deutlich größer als die Läsion sein, damit die Haut darum von der Folie vollständig abgedeckt wird.

### Vorbereitung der Behandlung

Der radioaktive Wirkstoff - im vorliegenden Beispielsfalle ¹⁸⁸Re - wird in einer sogenannten Karpule, die als Einwegartikel konzipiert ist und mit jeweils 0,3 ml Volumen beschickt ist, von der jeweiligen nuklearphysikalischen Anlage mit Aktivitäten bis zu 2,2 GBq pro Karpule zum Zeitpunkt der Anlieferung an der Klinik ausgeliefert. Aufgrund der nur geringen Halbwertszeit des ¹⁸⁸Re von etwa 16,7 Stunden muss vom Hersteller je nach Anlieferungsweg und Anlieferungszeit entsprechend höher kalibriert werden, um diese Radioaktivitätsmengen dann am Einsatzort zu gewährleisten.

Die Anlieferung erfolgt in einem abgeschirmten Transportbehälter und die Entnahme der Karpulen erfolgt erst kurz vor der Applikation. Die Karpule selbst enthält ¹⁸⁸Re in Form eines ¹⁸⁸Re₂S₇ als Dispersion in einer Acrylat-Wasser-TiO₂-basierten Matrixsuspension als Kolloid mit einem mittleren Teilchendurchmesser von im Beispielsfalle ca. 500 nm.

Vor Entnahme der radioaktiven Mischung aus der Karpule erfolgt zunächst eine erneute Vermischung der Karpule. Hierzu befindet sich in der Karpule ein Mischelement aus Metall, das von außen angetrieben in der Karpule auf und ab bewegt wird, so dass alle Komponenten der Matrix, insbesondere die TiO₂- und die ¹⁸⁸Re-Partikel, miteinander vermischt werden.

Danach wird ein an sich bekannter Applikator gemäß DE102009054388 mit jeder Karpule beladen. Der nächste Schritt ist die Bestimmung der tatsächlichen zum Applikationszeitpunkt in der beladenen Karpule enthaltenen Radioaktivitätsmenge. Die Bestimmung der Aktivität erfolgt zum Applikationszeitpunkt über ein einfaches Subtraktionsverfahren (Messung der Aktivität der Karpule vor der Behandlung, gleiche Messung nach Behandlung, resultierende Aktivität der Unterschiedsmessung wurde somit zur Behandlung des Patienten bzw. dessen Läsion eingesetzt).

Insbesondere wird der Applikator mit eingesetzter Karpule in einem speziellen Aktivimeter vermessen. Im Beispielsfalle handelt es sich um eine spezielle Schachtionisationskammer der Fa. MED Medizintechnik Dresden GmbH. Nach der Messung sind Applikator und Karpule bereit für die Behandlung.

### Behandlung - Aufbringen der Emissionsschicht-Matrix

Zur Behandlung wird die Karpule aktiviert, indem mit dem Applikator auf den Kopf der Karpule gedrückt wird. Dadurch wird die Karpule zusammengeschoben, und eine Nadel durchstößt eine Membran, welche den radioaktiven Inhalt zuvor zurück gehalten hat.

Nachdem die Schutzkappe von der Karpule abgenommen wurde, kann die Behandlung beginnen. Der behandelnde Arzt bestreicht die Trägerschicht-Folie über der Haut im zuvor markierten Bereich der Hautläsion mit der Polyacrylat-Matrix, so dass der ganze Bereich gleichmäßig bedeckt ist, um somit die Emissionsschicht, welche das ¹⁸⁸Re₂S₇ enthält zu bilden. Der Applikator ist zweigeteilt ausgeführt, wobei ein Teil wie ein dicker Stift geformt ist, so dass er gut handhabbar ist. Es ist außerdem eine Handabschirmung vorgesehen, die den Arzt vor radioaktiver Strahlung schützt. Die Dosierung der Matrix bzw. der Radioaktivitätsmenge erfolgt über den zweiten Teil des Applikators.

Die zähflüssige ¹⁸⁸Rhenium-haltige Polyacrylat-Matrix der Emissionsschicht härtet innerhalb von 10 bis 20 Minuten ohne wesentliche Schrumpfung aus. Im Anschluss an das Aufbringen der Emissionsschicht wird auf die noch leicht feuchte Emissionsschicht eine polyesterlaminierte Aluminiumfolie mit einer Dicke von ca. 100 µm als Abdeckschicht auf die distale Seite der Emissionsschicht aufgelegt, die so an der Polyacrylat-Matrix haftet.

Wenn die zu behandelnde Fläche bedeckt wurde und die Applikation beendet ist, wird die Schutzkappe wieder auf die Karpule gesteckt. Anschließend erfolgt erneut die Bestimmung der Aktivität der Karpule im Applikator mit Hilfe des oben erwähnten speziellen Aktivimeters. Aus der Differenz der Aktivitätsmessungen kann die aufgebrachte Radioaktivitätsmenge des ¹⁸⁸Re bestimmt werden. Mit der aufgebrachten Aktivität und der bedeckten Fläche kann die Behandlungsdauer berechnet werden. Die Behandlungsdauer liegt in der Regel zwischen 1 und 3 Stunden.

Für die Behandlungsdauer in Abhängigkeit von der Fläche des zu behandelnden Hautareals dienen folgende Tabellen 1 bis 4 als Orientierung:

**Tab. 1: Applikationszeit auf Basis einer Energiedosis von 50 Gy für eine erforderliche Behandlungstiefe von 300 µm**

| Behandlungsfläche [cm²] | Spezifische Radioaktivität [MBq/cm²] | Bestrahlungszeit [min] |
|---|---|---|
| 1 | 74 | 43 |
| 3 | 74 | 40 |
| 10 | 74 | 38 |

**Tab. 2: Applikationszeit auf Basis einer Energiedosis von 50 Gy für eine erforderliche Behandlungstiefe von 400 µm**

| Behandlungsfläche [cm²] | Spezifische Radioaktivität [MBq/cm²] | Bestrahlungszeit [min] |
|---|---|---|
| 1 | 74 | 49 |
| 3 | 74 | 45 |
| 10 | 74 | 43 |

**Tab. 3: Applikationszeit auf Basis einer Energiedosis von 50 Gy für eine erforderliche Behandlungstiefe von 500 µm**

| Behandlungsfläche [cm²] | Spezifische Radioaktivität [MBq/cm²] | Bestrahlungszeit [min] |
|---|---|---|
| 1 | 74 | 55 |
| 3 | 74 | 50 |
| 10 | 74 | 48 |

**Tab. 4: Applikationszeit auf Basis einer Energiedosis von 50 Gy für eine erforderliche Behandlungstiefe von 600 um**

| Behandlungsfläche [cm²] | Spezifische Radioaktivität [MBq/cm²] | Bestrahlungszeit [min] |
|---|---|---|
| 1 | 74 | 61 |
| 3 | 74 | 55 |
| 10 | 74 | 53 |

Bei der Behandlung von großen Läsionen hat sich herausgestellt, das es im Sinne einer minimalen Strahlenbelastung für den Patienten bei gleichzeitig effizienter Behandlung genügt, die Bestrahlungszeiten im Vergleich zu kleineren zu behandelnden Arealen zu reduzieren. Wie aus den obigen Tabellen 1 bis 4 ist ersichtlich, hat sich eine Reduktion der Bestrahlungszeit um ca. 12% bei einer Läsion mit einer Fläche von etwa 10 cm² im Vergleich zu einer von nur 1 cm² als günstig erwiesen.

Im Beispielsfalle einer histologisch als Basalzellkarzinom des nasolabialen Bereiches diagnostizierten Hautläsion mit einer Fläche von ca. 3 cm² lag die Behandlungsdauer bei ca. 55 Minuten (Energiedosis von 50 Gy für eine Behandlungstiefe von 600 µm, Spezifische Radioaktivität [MBq/cm²]¹⁸⁸Re, das aufgetragene Volumen betrug etwa 30 µl).

Die Karpule wird nach der Applikation in geeigneten abgeschirmten Abfallbehälter entsorgt. Aufgrund der nur geringen Halbwertszeit von 16,7 Stunden klingt die Radioaktivität des ¹⁸⁸Re dann innerhalb von etwa 10 Tagen ab.

### Entfernung der Emissionsschicht

Nach der Behandlungsdauer von 55 Minuten wird die Trägerschicht-Folie über der Hautläsion mit der aufgebrachten und mittlerweise eingetrockneten Emissionsschicht, die mit einer Abdeckfolie versehen wurde, mit einer langen Zange von der Haut des Patienten abgezogen und anschließend in einem abgeschirmten Abfallbehälter entsorgt.

Die Haut des Patienten wird nach der Behandlung gesäubert und auf strahlende Rückstände untersucht.

Aufgrund des erfindungsgemäßen Schichtaufbaus Haftschicht-Trägerschicht-Emissionsschicht und im Beispielsfalle noch zusätzlich einer Abdeckschicht wurde bei keinem Fall in etwa 500 Behandlungsfällen eine radioaktive Kontamination festgestellt.

Als eine mögliche Nebenwirkung wurde bei manchen Patienten eine leichte Rötung des behandelten Bereiches beobachtet. Nach Untersuchungen nach 2, 4 und 12 Wochen und dann in halbjährlichem Abstand zeigte sich in 85 % der Fälle eine Heilung ohne weitere notwendige Behandlung.

Lediglich in einigen Fällen war es erforderlich, eine zweite und ganz selten eine dritte Nachbehandlung durchzuführen, die dann sämtlich zum Erfolg führten.

Follow-up-Studien von bis zu 44 Monaten nach der Erstbehandlung zeigten eine 85-bis 90-prozentige Heilung bei BCC und SCC.

## Patentansprüche

1. Schichtaufbau zur epidermalen Radionuklidtherapie eines Patienten, mit:
- einer, aus Patientensicht proximalen, Haftschicht zum Anbringen des Schichtaufbaus auf einer zu behandelnden Hautoberfläche des Patienten, wobei eine Haftschicht aus einer Hautcreme und/oder einem Hautgel ausgenommen ist;
- einer aus Patientensicht in distaler Richtung über der Haftschicht angeordneten flexiblen, transparenten Trägerschicht-Folie; sowie
- wenigstens einer auf der Trägerschicht angeordneten radionuklidhaltigen Emissionsschicht;
wobei die Haftschicht und die Trägerschicht hinsichtlich ihrer Bestandteile und Dicke so ausgebildet sind, dass sie im Wesentlichen für α-, ß- und γ-Strahlung durchlässig sind,
wobei die Haftschicht vor dem Zusammenbau des Schichtaufbaus auf der Hautoberfläche gebildet wird, und
wobei die Haftschicht aus einem auf die Hautoberfläche aufsprühbaren Kunststofffilm gebildet ist,
**dadurch gekennzeichnet, dass**
der Kunststofffilm aus einem Sprühverband auf Basis von Polyurethanen, Polyacrylaten, insbesondere aus Poly(butylmethacrylat,methylmetacrylat) oder 2-Octylcyanacrylat gebildet ist;
die Trägerschicht-Folie ausgewählt wird aus der Gruppe bestehend aus:
Polyurethanfolie, Polyamidfolie, Polyolefinfolie, insbesondere Polyethylen- und Polypropylenfolie; und dass
die Emissionsschicht eine wasserbasierte Polyacrylatmatrix mit homogen darin verteiltem ¹⁸⁸Rheniumsulfid, insbesondere ¹⁸⁸Re₂S₇, ist.

2. Schichtaufbau nach Anspruch 1, **dadurch gekennzeichnet, dass** der Haftschicht-Sprühfilm eine Dicke von 1 bis 80 µm, insbesondere von 3 bis 60 µm aufweist.

3. Schichtaufbau nach Anspruch 1, **dadurch gekennzeichnet, dass** die Haftschicht auf der proximalen Seite der Trägerschicht angeordnet ist.

4. Schichtaufbau nach Anspruch 1, **dadurch gekennzeichnet, dass** die Trägerschicht zugleich Haftschicht ist, wobei die Trägerschicht als selbsthaftende Schicht ausgebildet ist.

5. Schichtaufbau nach einem der vorhergehenden Ansprüche, **dadurch gekennzeichnet, dass** die Trägerschicht als Folie mit einer Dicke von 15 bis 80 µm, insbesondere von 20 bis 60 µm, vorzugsweise von 30 bis 40 µm, ausgebildet ist.

6. Schichtaufbau nach Anspruch 1, **dadurch gekennzeichnet, dass** die Trägerschicht-Folie in ihrem äußeren Randbereich wenigstens eine Zone ohne Haftkraft aufweist.

7. Schichtaufbau nach einem der vorhergehenden Ansprüche, **dadurch gekennzeichnet, dass** die Emissionsschicht distal eine Abdeckschicht aufweist.

8. Schichtaufbau nach Anspruch 7, **dadurch gekennzeichnet, dass** die Abdeckschicht hinsichtlich ihrer Beschaffenheit und Dicke so ausgebildet ist, dass sie für die von den Radionukliden der Emissionsschicht emittierte α-, ß- und γ-Strahlung im Wesentlichen undurchlässig ist.

9. Schichtaufbau nach Anspruch 8, **dadurch gekennzeichnet, dass** die Abdeckschicht eine Polyester-, Polyethylen- oder Polypropylen-Folie oder eine mit Polyester, Polyethylen oder Polypropylen laminierte Aluminiumfolie ist, welche eine Dicke von 100 bis 200 µm aufweist.

## Claims

1. Layer structure for epidermal radionuclide therapy of a patient, comprising:
- an adhesive layer, which is proximal from the patient's perspective, for applying the layer structure to the surface of the patient's skin which is to be treated, an adhesive layer consisting of a skin cream and/or a skin gel being excluded,
- a flexible, transparent carrier layer film arranged above the adhesive layer in the distal direction from the patient's perspective; and
- at least one radionuclide-containing emission layer arranged on the carrier layer;
the adhesive layer and the carrier layer being designed in terms of their components and thickness such that they can be substantially penetrated by α, β and γ radiation, the adhesive layer being formed on the surface of the skin prior to the layer structure being assembled, and
the adhesive layer being made of a plastics film which can be sprayed onto the surface of the skin,
**characterised in that**
the plastics film is made of a spray-on bandage based on polyurethanes, polyacrylates, in particular of poly(butyl methacrylate,methyl methacrylate) or 2-octyl cyanoacrylate;
the carrier layer film is selected from the group that consists of:
polyurethane film, polyamide film, polyolefin film, in particular polyethylene film and polypropylene film; and **in that**
the emission layer is a water-based polyacrylate matrix having ¹⁸⁸rhenium sulfide, in particular ¹⁸⁸Re₂S₇, homogenously dispersed therein.

2. Layer structure according to claim 1, **characterised in that** the thickness of the adhesive layer spray-on film is from 1 to 80 µm, in particular from 3 to 60 µm.

3. Layer structure according to claim 1, **characterised in that** the adhesive layer is arranged on the proximal side of the carrier layer.

4. Layer structure according to claim 1, **characterised in that** the carrier layer is, at the same time, the adhesive layer, the carrier layer being designed as a self-adhesive layer.

5. Layer structure according to any one of the preceding claims, **characterised in that** the carrier layer is designed as a film having a thickness of from 15 to 80 µm, in particular of from 20 to 60 µm, preferably of from 30 to 40 µm.

6. Layer structure according to claim 1, **characterised in that** the carrier layer film comprises at least one non-adhesive area in its outer edge region.

7. Layer structure according to any one of the preceding claims, **characterised in that** the emission layer comprises a distal cover layer.

8. Layer structure according to claim 7, **characterised in that** the cover layer is designed in terms of its properties and thickness such that it substantially cannot be penetrated by the α, β and γ radiation emitted by the radionuclides of the emission layer.

9. Layer structure according to claim 8, **characterised in that** the cover later is a polyester film, a polyethylene film or a polypropylene film, or an aluminium foil laminated with polyester, polyethylene or polypropylene, which has a thickness of from 100 to 200 µm.

## Revendications

1. Structure en couches pour thérapie radionucléaire épidermique d'un patient, avec :
- une couche adhésive proximale du point de vue du patient, pour l'application de la structure en couches sur une surface cutanée à traiter du patient, dans laquelle une couche adhésive d'une crème cutanée et/ou d'un gel cutané est exclue,
- un film constituant une couche de support transparent, flexible, disposé du point de vue du patient en direction distale sur la couche adhésive ; ainsi que
- au moins une couche d'émission contenant des radionucléides, disposée sur la couche de support ;
dans laquelle la couche adhésive et la couche de support, en ce qui concerne leurs composants et leur épaisseur, sont réalisées de telle sorte qu'elles sont sensiblement perméables aux rayonnements α, β, et γ,
dans laquelle la couche adhésive est formée avant l'assemblage de la structure en couches sur la surface cutanée, et
dans laquelle la couche adhésive est formée à partir d'un film de matière plastique pouvant être pulvérisé sur la surface cutanée,
**caractérisée en ce que**
le film de matière plastique est formé à partir d'un aérosol à base de polyuréthanes, de polyacrylates, en particulier à partir de poly(butylméthacrylate, méthylmétacrylate) ou de 2-octylcyanacrylate ;
le film formant une couche de support est choisi dans le groupe consistant en : film de polyuréthane, film de polyamide, film de polyoléfine, en particulier de film de polyéthylène et de polypropylène ; et **en ce que**
la couche d'émission est une matrice de polyacrylate à base aqueuse avec du sulfure de rhénium-188 dispersé de façon homogène dans celle-ci, en particulier du ¹⁸⁸Re₂S₇.

2. Structure en couches selon la revendication 1, **caractérisée en ce que** le film aérosol formant une couche adhésive présente une épaisseur de 1 à 80 µm, en particulier de 3 à 60 µm.

3. Structure en couches selon la revendication 1, **caractérisée en ce que** la couche adhésive est disposée sur le côté proximal de la couche de support.

4. Structure en couches selon la revendication 1, **caractérisée en ce que** la couche de support est en même temps une couche adhésive, dans laquelle la couche de support est réalisée comme une couche auto-adhésive.

5. Structure en couches selon l'une des revendications précédentes, **caractérisée en ce que** la couche de support est réalisée comme un film avec une épaisseur de 15 à 80 µm, en particulier de 20 à 60 µm, de préférence de 30 à 40 µm.

6. Structure en couches selon la revendication 1, **caractérisée en ce que** le film formant une couche de support présente dans sa région périphérique externe au moins une zone sans pouvoir adhésif.

7. Structure en couches selon l'une des revendications précédentes, **caractérisée en ce que** la couche d'émission présente sur le plan distal, une couche de recouvrement.

8. Structure en couches selon la revendication 7, **caractérisée en ce que** la couche de recouvrement, en ce qui concerne sa constitution et son épaisseur, est réalisée de telle sorte qu'elle est sensiblement imperméable aux rayonnements α, β, et γ émis par les radionucléides de la couche d'émission.

9. Structure en couches selon la revendication 8, **caractérisée en ce que** la couche de recouvrement est un film de polyester, de polyéthylène ou de polypropylène ou un film d'aluminium stratifié avec du polyester, polyéthylène ou polypropylène qui présente une épaisseur de 100 à 200 µm.
